# EUROPÄISCHE PATENTSCHRIFT

(11) **EP 2 911 606 B1**
(45) Veröffentlichungstag und Bekanntmachung des Hinweises auf die Patenterteilung: **05.12.2018**
(21) Anmeldenummer: 13795686.8
(22) Anmeldetag: 26.10.2013
(51) Int. Cl.: A61B 90/11, A61B 17/34, A61B 90/00

(54) **INSTRUMENTENFÜHRUNG**
INSTRUMENT GUIDE
GUIDE D'INSTRUMENT

(30) Priorität: 26.10.2012 DE 202012010230 U
(43) Veröffentlichungstag der Anmeldung: 02.09.2015
(73) Patentinhaber: Isys Medizintechnik GmbH, 6370 Kitzbühel (AT)
(72) Erfinder: VOGELE, Michael, 86830 Schwabmünchen (DE)
(74) Vertreter: Fiener, Josef
(86) Internationale Anmeldenummer: PCT/EP2013/003224
(87) Internationale Veröffentlichungsnummer: WO 2014/063828

(56) Entgegenhaltungen:
- US-A1- 2006 149 147
- US-A1- 2012 184 956
- US-B1- 6 665 554

## Beschreibung

Die Erfindung betrifft eine Instrumentenführung, insbesondere zur Einführung von Punktionsnadeln bei Patienten.

Eine derartige Vorrichtung ist im Grundkonzept aus der DE 20 2004 003 646 des Erfinders bekannt, wobei sich diese Zielvorrichtung bei vielen operativen oder stereotaktischen Eingriffen mit genauer Ansteuerung von Punkten am oder im Körper bewährt hat. Vor allem durch die Einbeziehung moderner Computertechnologien, wie Computer-Tomographie (CT) oder Magnetresonanz-Tomographie, ist es möglich geworden, die erforderlichen Eintrittsorte, Eintrittstiefen und Eintrittsrichtungen der medizinischen Instrumente exakt festzulegen, so dass auch eine Zielvorrichtung zur Führung dieser Instrumente der erhöhten Genauigkeit gerecht wird. Mittels der z. B. durch CT ermittelten Patientendaten und Parameter soll dann ein Instrument an den definierten Zielpunkt am oder im Körper gebracht werden können. Ein spezieller Anwendungsfall mit Radiofrequenzablation (RFA) ist in "chemiereport.at 7/11, S. 42 beschrieben.

Wesentlich bei derartigen Zielvorrichtungen zur Führung medizinischer Instrumente sind eine hohe Zielgenauigkeit und eine schnelle Reproduzierbarkeit. Die stereotaktische Genauigkeit leidet bei wiederholten Eingriffen oftmals darunter, da für jedes Instrument die Vorrichtung umgestellt werden muss. So benötigen manche Behandlungen Punktionsnadeln mit unterschiedlichen Durchmessern, so dass nicht nur die Nadelhülsen ausgetauscht werde müssen, sondern sich wegen des unterschiedlichen Nadeldurchmessers auch oft die Zielachse verschiebt und somit nachjustiert werden muss. Dies gilt auch bei der Strahlen-Therapie, wobei sog. Pins oder Seeds direkt in das zu bestrahlende Tumorgewebe vorgeschoben werden. Durch den Einsatz computergestützter Navigationssysteme ist es zwar möglich geworden, entscheidende Verbesserungen auf diesem Gebiet zu erzielen, da auf dem Bildschirm anstatt der Lage der Sondenspitze nun die Lage der Nadelspitze im oder am Körper angezeigt wird. Die Anforderung einer schnellen und einfachen Reproduzierbarkeit bedarf jedoch noch Verbesserungen an der Zielvorrichtung, um die Verstellung in allen Raumachsen bzw. -ebenen exakt beibehalten zu können.

In der o.g. Schrift bzw. der davon abgeleiteten WO 2005/084565 wird eine präzise und variable Führung für medizinische Instrumente (u.a. Nadeln) mit übereinander angeordneten Stellantrieben ermöglicht, die in vorteilhafter Weise fernbedienbar sind. Die Instrumentenführung selbst wird dabei von zwei Mehrfachgelenken, insbesondere Kugelköpfen an den Vorderenden der Verstellarme gebildet, die jedoch relativ schwierig zu wechseln sind, zumal häufig unterschiedliche Nadeldicken erforderlich sind. Zudem sind in Anpassung an die jeweiligen Nadeldurchmesser mehrere Führungshülsen ("Inserts") nötig, die den Bereitstellungsaufwand erheblich steigern. Auch sind je nach Hersteller unterschiedliche Toleranzen vorhanden, so dass die Präzision und Feinfühligkeit des Nadelvorschubs durch leichtes "Verhaken" leiden kann. Dies gilt auch für den Fall einer schnellen Lösung der Nadel (wenn z. B. der Patient unruhig wird) und die Nadel bzw. das Instrument rasch aus der Halterung bereit werden muss.

US2006/0149147 offenbart eine Instrumentenführung mit veränderbarem Durchmesser unter Beibehaltung einer gemeinsamen zentralen Achse.

Daher liegt der Erfindung die Aufgabe zugrunde, eine einfach aufgebaute Instrumentenführung zu schaffen, die besonders variabel und bequem einzusetzen ist.

Diese Aufgabe wird gelöst durch eine Instrumentenführung mit den Merkmalen des Anspruches 1. Bevorzugte Ausführungen sind Gegenstand der Unteransprüche.

Durch die Ausgestaltung mit zwei miteinander gekoppelten Führungsbacken lässt sich eine exakt zentrische Verstellung des Nadelkanals erzielen. Zudem kann die Klemm- oder Führungskraft in vorteilhafter Weise eingestellt bzw. feinjustiert werden, um die Vorschubkräfte des jeweils eingesetzten Instruments an das jeweils zu behandelnde Gewebe anzupassen. Insbesondere beim wechselnden Einsatz von unterschiedlich dicken Nadeln ist diese Instrumentenführung von Vorteil, das hierbei das Zentrum des Nadelkanals genau auf einer Raumlinie verbleibt, also navigationstechnisch keine seitlichen Korrekturen der Zielachse vorgenommen werden brauchen. Somit kann der Wechsel von verschiedenen Nadeldicken besonders rasch und patientenschonend erfolgen.

Zudem ist damit die Positionierung zu einem Marker eindeutig definiert, um eine präzise Anpeilung von vorher, beispielsweise im CT festgelegten Eingriffs- oder Zielorten vorzunehmen, so dass eine erhebliche Reduzierung der Strahlenbelastung des OP-Personals und eine Erleichterung der in der Neurochirurgie üblichen Eingriffe erzielt wird. Ebenso kann im Notfall eine rasche und patientenschonende Befreiung des Instruments aus der Halterung erfolgen.

Zweckmäßig ist hierbei insbesondere die Befestigung der Halterung mit zwei Gelenkarmen an freien Enden von Verstellarmen der eingangs beschriebenen Vorrichtung. Es ist jedoch auch möglich, die Instrumentenführung bei Bedarf freihändig zu halten. Als Schnell-Rastverbindung sind insbesondere Einrastzapfen oder Klammern zur raschen und einfachen Ankoppelung an die Verstellarme vorgesehen. Gleiches gilt für einen Fortsatz an einer der Führungsbacken, der im Notfall bequem und rasch ergriffen werden kann. Die Führungsbacken können zudem leicht in den Grundkörper eingeführt werden und dort feinfühlig verstellt werden, insbesondere durch die leichtgängigen Schlitze bzw. Schrägführungen im Grundkörper. Die Nadel kann somit in exakt gleicher Ausrichtung wieder aufgenommen werden, insbesondere wenn diese gegen eine Nadel mit unterschiedlichem Durchmesser ausgetauscht wird.

Diese Instrumentenführung ermöglicht somit das Einführen der Instrumente in exakter Richtungseinstellung. Der präzisen Zielansteuerung dient auch die prismatische Form der Führungsbacken, da hierdurch bei allgemein zylindrischen Instrumenten (auch Nadeln) nur Linienberührungen auftreten, die einen feinfühligen Nadelvorschub sicherstellen. Zudem kann die Leichtgängigkeit durch reibungsvemindernde Beschichtungen oder die Verwendung von Wälzelementen (z. B. V-förmige Rollen) verbessert werden.

Nachfolgend wird ein bevorzugtes Ausführungsbeispiel anhand der Zeichnungen beschrieben. Hierbei zeigen:
Fig. 1 eine Seitenansicht einer Instrumentenführung mit zugehöriger Draufsicht in zwei Stellungen für unterschiedliche Nadeldurchmesser;
Fig. 2 eine Perspektivansicht der Instrumentenführung kurz vor Einsetzen in den Grundkörper; und
Fig. 3 eine Einzelansicht auf zwei ineinander gesteckte Führungsbacken.

Eine Instrumentenführung 1 ist hier mit einem klammerartigen Grundkörper 2 gezeigt, der mit Strichpunktlinien dargestellt ist. Der Grundkörper 2 ist bevorzugt mit einer nicht näher dargestellten Verstellvorrichtung über Verstellarme 3 und 4 verbunden, die vorzugsweise mit zwei Stellantrieben entsprechend dem eingangs diskutierten Stand der Technik justierbar sind. Hierdurch ist die Instrumentenführung 1 in mehreren Freiheitsgraden einstellbar.

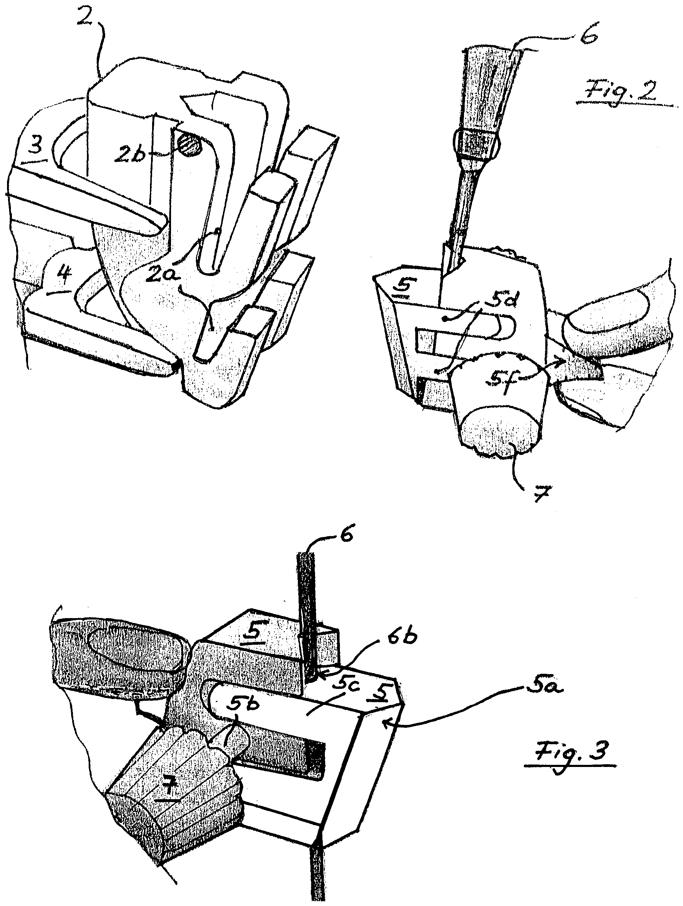

Innerhalb des Grundkörpers 2 sind zwei sich gegenüberliegende Führungsbacken 5 dargestellt, die zwischen sich eine Nadel 6 als Beispiel eines medizinischen Instruments aufnehmen. Die hier linke Führungsbacke 5 weist zwei Führungsbahnen 5a auf, die parallel zueinander in einem Winkel von ca. 15° zur Zentralachse 6a der Nadel 6 geneigt sind. Es kann jedoch auch nur eine Führungsbahn 5a ausreichen (vgl. Fig. 2 und 3). An der hier rechten Führungsbacke 5 sind zur Verstellung zwei Zapfen 5b vorgesehen, die in entsprechende Führungsschlitze 2a am Grundkörper 2 eingreifen, wobei diese Schlitze 2a die gleiche Neigung wie die Führungsbahnen 5a aufweisen, nur in spiegelbildlicher Ausrichtung zur Mittelebene, die die Zentralachse 6a enthält. Hierdurch wird eine synchrone, zentrische Verstellung in Bezug auf die Zentralachse 6a erzielt, wobei zur Koppelung der beiden Führungsbacken 5 wenigstens ein Mitnehmer 5c, hier in Form von zwei Stiften 5d vorgesehen ist/sind. Hierdurch wird beim Niederdrücken einer Führungsbacke 5 (insbesondere hier die gut zugängliche rechte) die andere Führungsbacke 5 mitgenommen und so der exakt gleiche Abstand von der Zentralachse 6a sichergestellt, so dass bei einem Austausch der Nadel 6 (hier in Draufsicht links mit relativ großem Durchmesser) zu einer dünneren Nadel 6 (hier rechts unten in Draufsicht dargestellt) eine exakte Ausrichtung in der Zentralachse 6a beibehalten wird, da beide Führungsbacken 5 synchron zur Zentralachse 6a hin verstellt werden. Diese Mitnehmer-Kopplung der Führungsbacken 5 kann auch mit Exzentern, Parallelogrammen oder ähnlichen Mechanismen erzielt werden.

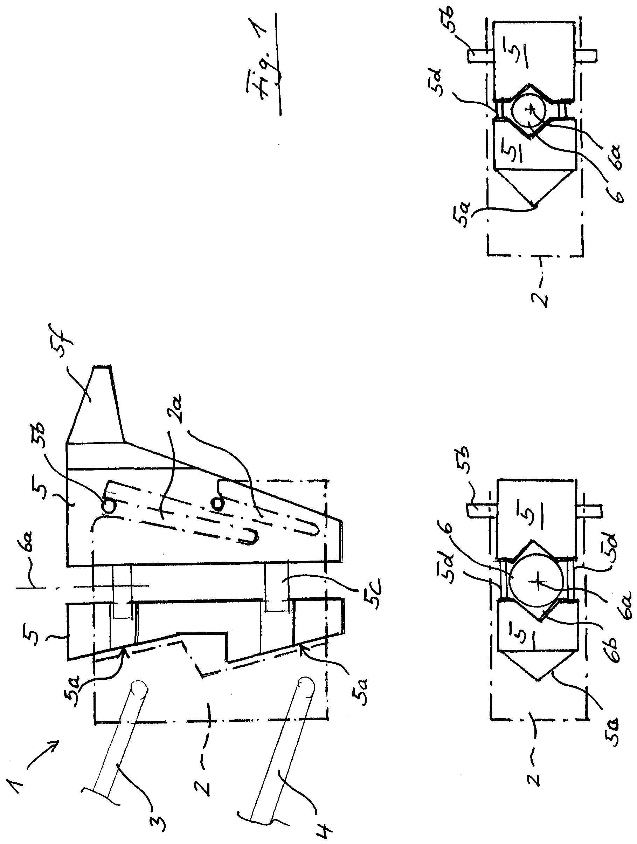

Wie aus den zugehörigen Draufsichten in Fig. 1 dargestellt ist, weisen die Führungsbacken 5 zur Nadel 6 hin eine prismatische Form oder jeweils V-förmige Nut auf, um so einen Nadelkanal 6b zu bilden. Die Rückseite der hier linken Führungsbacke 5 bzw. Führungsbahn 5a ist pfeilförmig gestaltet, um so eine entsprechend hohe Seitenführung und Exaktheit bei der Verstellung zu gewährleisten (vgl. auch Fig. 2 und 3). Die vorstehend genannten Flächen können eine reibungsarme Beschichtung aufweisen, um so die Leichtgängigkeit zu erhöhen. Der Nadelkanal 6b kann auch durch Rollen oder V-Förmige Walzen begrenzt sein, um so den Vorschub von größeren Instrumenten (wie Bohrern in der Knochenchirurgie) zu erleichtern. Der Grundkörper 2 und die Führungsbacken 5 sind bevorzugt aus röntgentransparenten Kunststoffen hergestellt.

In Fig. 2 und 3 sind insbesondere die Mitnehmer 5c in Form der zwei Stifte 5d ersichtlich, ebenso Feststellmuttern 7 auf den Zapfen 5b. Durch Festziehen kann somit der Grad der Klemmkraft und damit die Leichtgängigkeit der Nadel 6 angepasst werden. Zudem ist ein Fortsatz 5f ersichtlich, mit dem eine Führungsbacke 5 leicht zu greifen ist (vgl. den dargestellten Daumen bzw. Finger). Damit können die Führungsbacken 5 einfach in den Grundkörper 2 eingesetzt werden, wie dies in Fig. 2 angedeutet ist.

Da der Fortsatz 5f entgegengesetzt zu den Verstellarmen 3 und 4 angeordnet ist, kann im Notfall diese "äußere" Führungsbacke 5 entlang dem Nadelkanal 6b rasch entnommen werden, so dass dann auch die Nadel 6 frei wird. Diese Führungsbacke 5 kann auch als Klammer ausgebildet sein, die durch kräftiges Zusammendrücken zwischen Daumen und Finger aus dem Rasteingriff mit dem Grundkörper 2 frei gegeben wird.

In Fig. 2 sind außen am Grundkörper 2 mehrere Vertiefungen erkennbar, die der Aufnahme von Markern 2b (hier nur einer angedeutet) zur Referenzierung bzw. Navigation dienen. Die Marker 2b können auch an einem separaten Gestänge vorgesehen sein, das angrenzend zum Grundkörper 2 befestigt wird. Zudem können an dem Grundkörper 2 auch Vorschubantriebe (z. B. Feingewindespindeln) zum Vorschub der Instrumente bzw. der Nadel 6 entlang der Zentralachse 6a aufgesetzt sein.

## Patentansprüche

1. Instrumentenführung, insbesondere zur Einführung von Punktionsnadeln bei Patienten, wobei die Instrumentenführung (1) einen Grundkörper (2) aufweist, der bevorzugt an zwei Verstellarmen (3, 4) in mehreren Achsen gelenkig gelagert ist, wobei die Instrumentenführung (1) unter Bildung eines Nadelkanals (6b) mit einer Zentralachse (6a) zwei oder mehr Führungsbacken (5) aufweist, die miteinander gekoppelt verstellbar sind, **dadurch gekennzeichnet, dass**
wenigstens einer der Führungsbacken (5) mindestens einen seitlich abstehenden Zapfen (5b) zur Ankoppelung an den Grundkörper (2) aufweist, wobei an einem anderen Führungsbacken (5) wenigstens eine geneigte Führungsbahn (5a) und am Grundkörper (2) ein gleich geneigter, spiegelbildlich zur Mittelebene ausgerichteter Führungsschlitz (2a) vorgesehen sind, in dem der Zapfen (5b) zur zentrischen Verstellung des Aufnahmedurchmessers des Nadelkanals (6b) unter Beibehaltung der Zentralachse (6a) geführt ist.

2. Instrumentenführung nach Anspruch 1, **dadurch gekennzeichnet, dass** die Führungsbacken (5) eine prismatische Form mit Ausbildung eines doppelt V-förmigen Nadelkanals (6b) aufweisen.

3. Instrumentenführung nach Anspruch 1 oder 2, **dadurch gekennzeichnet, dass** wenigstens einer der Führungsbacken (5) zum Grundkörper (2) hin eine Pfeilform aufweist.

4. Instrumentenführung nach einem der Ansprüche 1 bis 3, **dadurch gekennzeichnet, dass** wenigstens einer der Führungsbacken (5) als Klammer ausgebildet ist.

5. Instrumentenführung nach einem der Ansprüche 1 bis 4, **dadurch gekennzeichnet, dass** der Zapfen (5b) eine Feststellmutter (7) trägt.

6. Instrumentenführung nach einem der Ansprüche 1 bis 5, **dadurch gekennzeichnet, dass** einer der Führungsbacken (5) einen abstehenden, leicht greifbaren Fortsatz (5c) zur Schnelllösung des Führungsbackens (5) aufweist.

7. Instrurnentenführung nach einem der Ansprüche 1 bis 6, **dadurch gekennzeichnet, dass** die Führungsbacken (5) mit wenigstens einem Mitnehmer (5c) gekoppelt sind, insbesondere in Form von zwei Stiften (5d).

8. Instrumentenführung nach einem der Ansprüche 1 bis 7, **dadurch gekennzeichnet, dass** der Grundkörper (2) oder ein daran angrenzendes Element wenigstens einen Marker (2b) zur Referenzierung für Bildgebung oder Navigation aufweist.

9. Instrumentenführung nach einem der Ansprüche 1 bis 8, **dadurch gekennzeichnet, dass** die Klemmkraft der Führungsbacken (5) einstellbar ist.

10. Instrumentenführung nach einem der Ansprüche 1 bis 9, **dadurch gekennzeichnet, dass** der Grundkörper (2) oder ein daran angeschlossenes Element Skalierungen und/oder Anschläge zur Einstellung der Nadel-Eindringtiefe aufweist.

11. Instrumentenführung nach einem der Ansprüche 1 bis 10, **dadurch gekennzeichnet, dass** die Führungsbacken (5) zum Nadelkanal (6b) hin reibungsmindernd ausgebildet sind, insbesondere mit Rollen oder V-förmigen Walzen.

## Claims

1. Instrument guide, especially for inserting of puncture needles into patients, wherein the instrument guide (1) comprises a base body (2) being preferably pivotably mounted in plural axes on two adjusting arms (3, 4), wherein the instrument guide (1) forms a needle channel (6b) having a central axis (6a) and includes two or more guide jaws (5) coupled to one another in an adjustable manner,
**characterized in that**
at least one of the guide jaws (5) has at least one laterally projecting pin (5b) for coupling to the base body (2), wherein another guide jaw (5) includes at least one inclined guide way (5a) and the base body (2) provides a guide slot (2a), inclined in the same way, but in mirror image alignment to the middle plane for guiding the pin (5b) on centric adjustment of the outer diameter of the needle channel (6b), thus maintaining the central axis (6a).

2. Instrument guide according to claim 1, **characterized in that** the guide jaws (5) have a prismatic shape with formation of a double V-shaped needle channel (6b).

3. Instrument guide according to claim 1 or 2, **characterized in that** at least one of the guide jaws (5) has an arrow shape directed towards the base body (2).

4. Instrument guide according to one of claims 1 to 3, **characterized in that** at least one of the guide jaws (5) is formed as a clamp.

5. Instrument guide according to one of claims 1 to 4, **characterized in that** the pin (5b) carries a locking nut (7).

6. Instrument guide according to one of claims 1 to 5, **characterized in that** one of the guide jaws (5) has a protruding, slightly tangible extension (5c) for quick release of the guide jaw (5).

7. Instrument guide according to one of claims 1 to 6, **characterized in that** the guide jaws (5) are coupled with at least one follower (5c), especially in the form of two pins (5d).

8. Instrument guide according to one of claims 1 to 7, **characterized in that** the base body (2) or a structure adjacent thereto has at least one marker element (2b) for image referencing or navigation.

9. Instrument guide according to one of claims 1 to 8, **characterized in that** the clamping force of the guide jaws (5) is adjustable.

10. Instrument guide according to one of claims 1 to 9, **characterized in that** the base body (2) or an element connected thereto has a scaling and/or stop for adjustment of the needle insertion depth.

11. Instrument guide according to one of claims 1 to 10, **characterized in that** the guide jaws (5) for the needle channel (6b) are formed in a friction-reducing manner, especially with cylindrical rollers or V-shaped rollers.

## Revendications

1. Guide-instruments destiné, en particulier, à l'introduction d'aiguilles de ponction auprès de patients, lequel guide-instruments (1) est pourvu d'un corps de base (2) monté de préférence, sur deux bras de réglage (3, 4), de manière articulée suivant plusieurs axes, ledit guide-instruments (1) étant doté, en vue de former un canal (6b) de passage d'aiguilles présentant un axe central (6a), de mâchoires de guidage (5) au nombre de deux ou plus, mutuellement couplées avec faculté de réglage,
**caractérisé par le fait**
**qu'**au moins l'une des mâchoires de guidage (5) est munie d'au moins un téton (5b) faisant saillie dans le sens latéral, en vue du couplage au corps de base (2), sachant qu'au moins une piste inclinée de guidage (5a) est prévue sur une autre mâchoire de guidage (5) et qu'il est prévu, dans ledit corps de base (2), une fente de guidage (2a) qui présente une inclinaison identique, est orientée spéculairement par rapport au plan médian, et dans laquelle ledit téton (5b) est guidé en vue du réglage centré du diamètre de réception du canal (6b) de passage d'aiguilles, avec conservation de l'axe central (6a).

2. Guide-instruments selon la revendication 1, **caractérisé par le fait que** les mâchoires de guidage (5) possèdent une forme prismatique, avec formation d'un canal (6b) de passage d'aiguilles configuré en forme de V double.

3. Guide-instruments selon la revendication 1 ou 2, **caractérisé par le fait qu'**au moins l'une des mâchoires de guidage (5) revêt la forme d'une flèche pointant en direction du corps de base (2).

4. Guide-instruments selon l'une des revendications 1 à 3, **caractérisé par le fait qu'**au moins l'une des mâchoires de guidage (5) est réalisée sous la forme d'une pince.

5. Guide-instruments selon l'une des revendications 1 à 4, **caractérisé par le fait que** le téton (5b) porte un écrou de blocage (7).

6. Guide-instruments selon l'une des revendications 1 à 5, **caractérisé par le fait que** l'une des mâchoires de guidage (5) comporte un appendice (5c) en saillie, aisément saisissable, en vue de la dissociation rapide de ladite mâchoire de guidage (5).

7. Guide-instruments selon l'une des revendications 1 à 6, **caractérisé par le fait que** les mâchoires de guidage (5) sont couplées à au moins un organe d'entraînement (5c) revêtant, en particulier, la forme de deux broches (5d).

8. Guide-instruments selon l'une des revendications 1 à 7, **caractérisé par le fait que** le corps de base (2), ou un élément limitrophe de ce dernier, est muni d'au moins un repère (2b) affecté au référencement à des fins d'imagerie ou de navigation.

9. Guide-instruments selon l'une des revendications 1 à 8, **caractérisé par le fait que** la force de coincement des mâchoires de guidage (5) est réglable.

10. Guide-instruments selon l'une des revendications 1 à 9, **caractérisé par le fait que** le corps de base (2), ou un élément rattaché à ce dernier, est muni de graduations et/ou de butées dévolues au réglage de la profondeur de pénétration des aiguilles.

11. Guide-instruments selon l'une des revendications 1 à 10, **caractérisé par le fait que** les mâchoires de guidage (5) sont de réalisation atténuant le frottement en direction du canal (6b) de passage d'aiguilles, notamment par des galets ou des rouleaux configurés en forme de V.
